# EUROPÄISCHE PATENTSCHRIFT

(11) **EP 3 341 078 B1**
(45) Veröffentlichungstag und Bekanntmachung des Hinweises auf die Patenterteilung: **21.08.2019**
(21) Anmeldenummer: 16763195.1
(22) Anmeldetag: 26.08.2016
(51) Int. Cl.: A61N 2/00, A61N 2/02

(54) **MAGNETFELDGENERATOR ALS NATURFELDSIMULATOR**
MAGNETIC FIELD GENERATOR AS A NATURAL FIELD SIMULATOR
GÉNÉRATEUR DE CHAMP MAGNÉTIQUE EN TANT QUE SIMULATEUR DE CHAMP NATUREL

(30) Priorität: 28.08.2015 DE 102015011092
(43) Veröffentlichungstag der Anmeldung: 04.07.2018
(73) Patentinhaber: Feucht, Peter, 12247 Berlin (DE)
(72) Erfinder: Feucht, Peter, 12247 Berlin (DE)
(74) Vertreter: Liebl, Thomas
(86) Internationale Anmeldenummer: PCT/EP2016/001447
(87) Internationale Veröffentlichungsnummer: WO 2017/036585

(56) Entgegenhaltungen:
- EP-A1- 1 170 032
- WO-A1-00/61217
- WO-A1-89/05673
- JP-A- 2008 131 708
- US-A1- 2005 182 287
- US-A1- 2012 296 150

## Beschreibung

Die Erfindung betrifft einen Magnetfeldgenerator als Naturfeldsimulator nach dem Oberbegriff des Anspruchs 1.

Solche Magnetfeldgeneratoren als Naturfeldsimulatoren sind allgemein bekannt mit einem am Körper tragbaren Gehäuse, das eine Magnetspule auf einem Magnet-Eisenkern zur Felderzeugung, eine Elektronik auf einer Elektronikplatine für eine Impulserzeugung und eine Hochleistungsbatterie zur Stromversorgung enthält.

Die technische Funktion sowie die Wirkung ist bei den bekannten Magnetfeldgeneratoren weitgehend gleich und wird auch prinzipiell beim vorliegenden erfindungsgemäßen Magnetfeldgenerator verwendet. Der Vollständigkeit halber wird die technische Funktion in Verbindung mit den verwendeten Bauteilen kurz erläutert:
Das natürliche Erdmagnetfeld weist zusätzlich zum statischen Magnetfeld ein schwaches Wechselfeld mit der sogenannten Schuhmann-Frequenz von zirka 8Hz sowie zusätzlich noch sogenannte hochfrequente Schönwetter-Sferics insbesondere im Bereich von 10kHz auf. Diese Magnetfelder werden bei den bekannten Naturfeldsimulatoren mit einer Magnetspule auf einem Magnet-Eisenkern und gegebenenfalls zusammen mit einem Permanentmagneten künstlich erzeugt, wobei eine 10kHz-Schwingung aus der Energierückgewinnung aus der Magnetspule durch abruptes Abschalten erzeugt werden kann.

Das natürliche Erdmagnetfeld insbesondere in Verbindung mit dem Schuhmann-Wechselfeld und dem Schönwetter-Sferics soll eine positive Wirkung auf den Menschen haben. Durch Umwelteinflüsse insbesondere in Städten sind diese Naturfelder nur abgeschwächt vorhanden und werden durch Störfelder elektrischer Anlagen und Sender aller Art weitgehend verdeckt. Mit den bekannten Naturfeldsimulatoren und auch mit dem erfindungsgemäßen Naturfeldsimulator soll das natürliche Magnet-Wechselfeld körpernah zum Ausgleich simuliert werden, wobei ein Naturfeldsimulator bevorzugt an einem Halsband vor der Brust in Höhe der Thymusdrüse getragen werden soll. Angenehm und gegebenenfalls unauffällig zu tragen ist somit ein solcher Naturfeldsimulator nur dann, wenn er wenig Gewicht hat, klein ist und nicht als technisches Gerät erkennbar in Erscheinung tritt.

Bei bekannten, auf dem Markt befindlichen Naturfeldsimulatoren Typ NFS8 (Hersteller: FM Elektronik, Berlin) ist die Anordnung der Einzelbauteile sandwichartig übereinander ausgeführt mit einer zentralen Elektronikplatine auf deren einer Seite die Magnetspule mit einem Magnet-Eisenkern aus Vollmaterial zusammen mit Elektronikelementen angeordnet ist. Auf der gegenüberliegenden Seite der Elektronikplatine ist eine Halterung für zwei Lithium-Knopfbatterien angebracht. Diese Baugruppe ist in einem zweischaligen Gehäuse mit den Abmessungen 56 x 31 x 24 mm enthalten, wobei aus dem Gehäuse noch ein Aufhänger mit einer Öse ragt. Das Gehäuse ist ersichtlich so groß und so schwer, dass dieser Naturfeldsimulator nicht unauffällig und/oder in der Art eines Halsband-Schmuckstücks getragen werden kann, sondern als technisches Gerät am Träger auffällt und zu möglicherweise ungewollten Fragen für den Träger Anlass gibt.

Ein anderer bekannter Naturfeldsimulator Typ LFS (Vertrieb: ORTUS Buch und Marketing Verlag) hat die Gehäuseform einer flachen Scheibe mit einem Scheibendurchmesser von 65 mm und einer Scheibendicke von 20 mm. Die relativ geringe Dicke der Gehäusescheibe wird dadurch erreicht, dass eine kreisförmige Elektronikplatine eine zentrale Längsausnehmung aufweist, in der die Magnetspule mit ihrem Magneteisenkern aus Vollmaterial angeordnet ist. Auch dieser Naturfeldsimulator ist so groß und schwer, dass er weder unauffällig noch bequem am Halsband zu tragen ist.

Ein weiter bekannter, gattungsgemäßer Naturfeldsimulator in einem am Körper tragbaren Gehäuse (US 2005/182287 A1) weist eine Magnetspule auf einem Magnet-Eisenkern zur Felderzeugung, sowie eine Elektronik auf einer Elektronikplatine für eine Impulserzeugung und eine Hochleistungsbatterie zur Stromerzeugung auf. Der Magnet-Eisenkern ist zudem als Hohlkörper aus elektrisch leitfähigem Material ausgebildet, wobei die Magnetspule auf den Magnet-Eisenkern-Hohlkörper gewickelt ist.

Konkret ist hier die Elektronik als Mikrochip an der offenen Stirnseite des Magnet-Eisenkern-Hohlkörpers angeordnet (Abbildung 11C). Nachteilig ergibt sich durch diese Anordnung, dass eine wirksame Abschirmung der Elektronik von außen gegen eine ungünstige Beeinflussung durch das Magnetfeld der Magnetspule nicht erreichbar ist.

Zudem ist durch diese Anordnung keine wirksame Abschirmung der Elektronik nach außen erreichbar, um eine ungünstige Abstrahlung von Störimpulsen aus der Elektronik zu verhindern.

Aufgabe der Erfindung ist es daher, einen gattungsgemäßen Naturfeldsimulator so weiterzubilden, dass ungünstige Einflüsse auf die Elektronik und zugleich von der Elektronik ausgehende ungünstige Einflüsse verhindert oder zumindest weitestgehend reduziert werden.

Diese Aufgabe wird dadurch gelöst, dass die Elektronikplatine mit den Elektronikbauteilen zumindest teilweise im Magnet-Eisenkern-Hohlkörper aufgenommen ist.

Der Eisenkern-Hohlkörper kann hier bezüglich der darin aufgenommenen Elektronik besonders vorteilhaft zwei Funktionen erfüllen:
1. die im Eisenkern-Hohlkörper zumindest teilweise aufgenommene Elektronik wird vom Magnetfeld der Magnetspule hinreichend abgeschirmt, und
2. durch das elektrisch leitfähige Material des Eisenkern-Hohlkörpers wird eine ungünstige Abstrahlung von Störimpulsen aus der darin aufgenommenen Elektronik verhindert.

In einer konkreten Ausführung kann der Eisenkern-Hohlkörper ein beidseitig offenes Eisenkern-Blechrohr bestimmter Länge sein, in das eine längere Elektronik-Platine mit beidseitigen Platinenüberständen eingesetzt ist.

In einer weiteren Ausgestaltung kann dann ergonomisch günstig und funktionell an einem Platinenüberstand ein durch einen Durchbruch des Gehäuses ragender Schalter, vorzugsweise als Drehschalter-Nippel zur Aktivierung und Deaktivierung des Naturfeldsimulators angeordnet sein, das insbesondere eine Bohrung zur Befestigung an einem Halsband aufweisen kann.

Zudem kann am anderen Platinenüberstand, außerhalb des Bereichs des Eisenkern-Blechrohrs eine betriebsanzeigende Leuchtdiode angeordnet sein. Diese soll dann hinter einer transparent abgedeckten, vorzugsweise durch einen Kristall abgedeckten Gehäuseöffnung als Lichtaustrittsöffnung liegen. Der Kristall kann sternförmig für einen geführten Lichtaustritt geschliffen sein und in einer hochwertigen Ausführung gegebenenfalls ein Edelstein sein.

Zudem kann dieser Platinenüberstand endseitig konkav, gegebenenfalls mit Kontaktfedern ausgerüstet zu einer flächengleichen Aufnahme einer Knopfbatterie ausgebildet sein. Insgesamt wird damit eine insgesamt längliche Form der Elektronikplatine erreicht, die in geeigneter Weise in den Eisenkern-Hohlkörper, insbesondere in ein Eisenkern-Blechrohr eingesetzt werden kann.

In einer weiteren vorteilhaften Ausgestaltung soll der Eisenkern-Hohlkörper als Eisenkern-Blechrohr aus zwei, vorzugsweise flachen Schalenteilen gebildet sein, deren jeweils einander gegenüberliegende Schalenlängsränder durch wenigstens einen in Längsrichtung zum Magnetfeld verlaufenden Spalt beabstandet sind. Der eine oder mehrere isolierende Spalte behindern den Magnetfluss nicht aber unterbrechen vorteilhaft einen durch die Magnetspule induzierten Stromfluss (Kurzschlussstrom).

Zur Realisierung der Spalte kann die Elektronikplatine einfach seitlich abstehende Nocken als Platinenfortsätze aufweisen, die jeweils zwischen die Schalenränder ragen. Zudem können die Schalenränder jeweils im Nockenbereich eine Haltekontur für die dazwischenliegenden Nocken zur Fixierung der Elektronikplatine aufweisen, wobei die Schalenteile über die Elektronikplatine verbunden, insbesondere dort verlötet sind. Für das Eisenkern-Blechrohr können einfach zwei Schalenteile als Gleichteile verwendet werden, die untereinander und formschlüssig über die in den Halbschalen angeordnete Elektronikplatine verbunden, vorzugsweise verlötet werden.

Es ist auch möglich, zwei unterschiedliche Schalenteile zu verwenden, insbesondere ein flaches Schalenteil und ein höheres Schalendeckelteil, wenn insbesondere die Elektronikteile auf einer Fläche der Elektronikplatine Platz finden und dadurch die Unterseite der Elektronikplatine nicht bestückt ist. Die Elektronikplatine kann dann in das untere flache Schalenteil eingesetzt werden und mit dem höheren Deckelschalenteil zur Ausbildung des Eisenkern-Blechrohrs abgedeckt werden.

Jedenfalls bildet der Eisenkern als Hohlkörper mit zumindest teilweise darin aufgenommenen Elektronikplatine eine Baugruppe, die auch den Wickelkörper für die aufzubringende Magnetwicklung als Magnetspule bildet.

Die Wickelenden der Spule können auf der Wickelmaschine für unrunde Wicklungen direkt an Kontaktstellen auf die Elektronikplatine gelötet werden.

Als Hochleistungsbatterie kann eine Lithium-Knopfzelle verwendet werden, die aus einer magnetisch leitenden Metall-Legierung besteht. Diese ist vorteilhaft in Richtung der magnetischen Achse der Magnetspule angeordnet und dient somit der effektiven Vergrößerung der Magnetwirkung (Streufeld der Magnetspule).

Das Gehäuse soll eine durch eine Verschlussklappe lösbar verschlossene Öffnung als Batteriefach aufweisen, durch die bei der Montage auch die Baugruppe einführbar und fixierbar ist.

Anhand einer Zeichnung wird die Erfindung weiter erläutert.

Es zeigen:
- Fig. 1: eine Draufsicht auf einen Magnetfeldgenerator als Naturfeldsimulator,
- Fig. 2: eine Seitenansicht des Naturfeldsimulators nach Fig. 1,
- Fig. 3: eine Draufsicht auf die interne Baugruppe des Naturfeldsimulators nach Fig. 1,
- Fig. 4: eine Schnittdarstellung entlang der Linie A-A nach Fig. 3 in zwei Ausführungsformen,
- Fig. 5: eine vergrößerte Darstellung eines zweischaligen EisenkernBlechrohrs,
- Fig. 6: eine unbestückte Elektronikplatine, und
- Fig. 7: einen Schnitt entlang der Linie B-B nach Fig. 5.

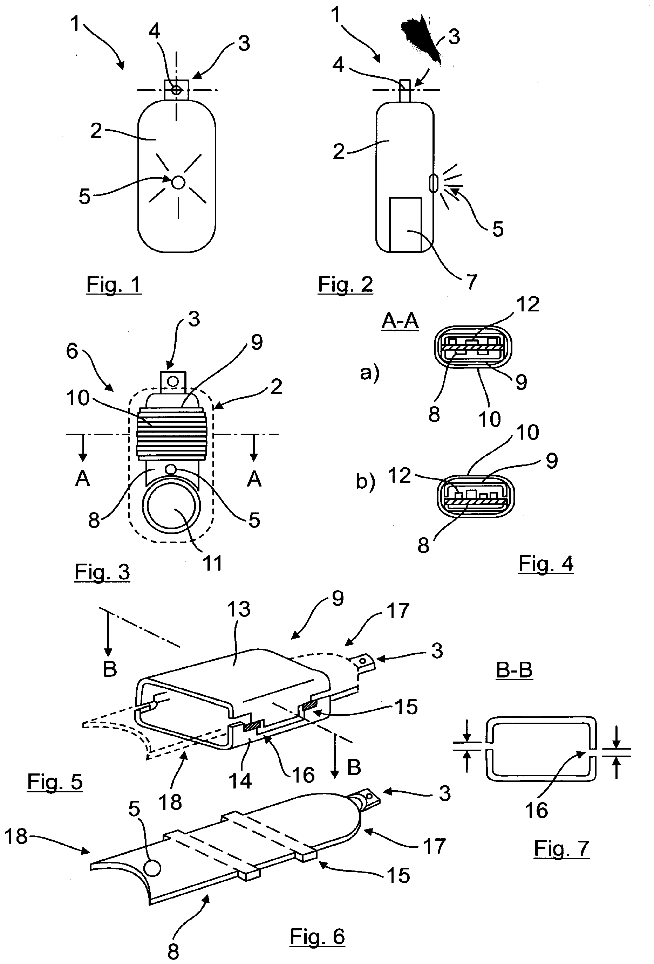

In den Fig. 1 und 2 sind eine Draufsicht und eine Seitenansicht eines Magnetfeldgenerators als Naturfeldsimulator 1 etwa in natürlicher Größe dargestellt. Aus dem Gehäuse 2 ragt an der Oberseite ein Drehschalter 3, der zugleich mit einer Öse 4 als Aufhänger verwendbar ist, so dass der Naturfeldsimulator 1 an einem Halsband auf der Brust vor einer Thymusdrüse getragen werden kann.

An der Frontseite des Naturfeldsimulators 1 ist eine mit einem Kristall 5 abgedeckte Lichtaustrittöffnung angeordnet, hinter der eine Leuchtdiode 5 einer Baugruppe 6 liegt (Fig. 3).

Zudem weist das Gehäuse 2 eine durch eine Verschlusskappe 7 lösbar abgedeckte Öffnung als Batteriefach auf, durch die auch die Baugruppe (6) bei der Montage eingeführt und fixiert werden kann.

Die Baugruppe 6 besteht im Wesentlichen aus einer Elektronikplatine 8, einem Magnet-Eisenkern als Eisenkern-Blechrohr 9, einer Magnetspule 10, die auf das Eisenkern-Blechrohr 9 gewickelt ist und einer Lithium-Knopfbatterie 11. Die Baugruppe 6 ist im montierten Zustand in das Gehäuse 2 eingesteckt und füllt dieses weitgehend aus, wie mit der strichlierten Linie als Gehäuse 2 angedeutet. Der aus dem Gehäuse 2 herausragende Drehschalter 3 ist an der Elektronikplatine 8 befestigt.

Fig. 4a zeigt einen Querschnitt im Bereich des Eisenkern-Blechrohrs 9 und der Magnetspule 10 mit einer Elektronikplatine 8 die beidseitig mit schematisch angedeuteten Elektronikelementen 12 bestückt ist. Die Elektronikplatine 8 ist in Fig. 4a entsprechend in der Längsmitte des Eisenblechrohrs 9 angeordnet, wie dies detailliert in den vergrößerten Darstellungen der Fig. 5 bis 7 mit zwei gleichen Blechschalenteilen 13, 14 gezeigt ist.

Wenn die Elektronikplatine 8 wie in Fig. 4b dargestellt nur einseitig mit Elektronikelementen 12 bestückt ist, kann es zweckmäßig sein das untere Blechschalenteil flacher auszubilden und die Elektronikplatine 8 tiefer zu setzen, wodurch gegebenenfalls eine weitere Volumenreduzierung möglich sein kann.

In Fig. 5 ist das Eisenkern-Blechrohr 9 bestehend aus zwei Blechschalenteilen 13, 14 vergrößert und mit weiteren Details dargestellt. Zudem ist in Fig. 6 die (unbestückte) Elektronikplatine 8, die zwischen den Blechschalenteilen 13, 14 eingesetzt und gehalten wird, vergrößert gezeigt. Dazu sind an der Elektronikplatine 8 an beiden Längsrändern jeweils zwei gegeneinander versetzte Nocken ausgeformt, die zwischen den Längsrändern der Blechschalenteile 13, 14 liegen, wodurch beidseitig zwischen den Blechschalenteilen 13, 14 zur elektrischen Isolierung ein Luftspalt 16 gebildet ist, wie dies insbesondere aus dem Querschnitt entlang der Linie B-B nach Fig. 7 ersichtlich ist. Zudem weisen die Längsrändern der Blechschalenteile 13, 14 im Bereich der Nocken 15 eine gestufte Haltekontur auf, wodurch die Elektronikplatine 8 in der fertigen Baugruppe 6 lagerichtig gehalten und fixiert ist.

Das Eisenkern-Blechrohr 9 aus den beiden Blechschalenteilen 13, 14 ist kürzer als die Elektronikplatine 8, welche beidseitig mit Platinenüberständen 17, 18 die Blechschalenteile 13, 14 in Längsrichtung überragt. Dabei ist am oberen Platinenüberstand 17 der aus dem Gehäuse 2 nach oben abragende Drehschalter 3 angebracht. Am unteren Platinenüberstand 18 ist die Leuchtdiode 5 angeordnet, die dadurch nicht von den Blechschalenteilen 13, 14 abgedeckt ist und hinter der Lichtaustrittsöffnung am Gehäuse 2 liegt, die optisch ansprechend durch einen geschliffenen Kristall ist.

## Patentansprüche

1. Magnetfeldgenerator als Naturfeldsimulator (1) in einem am Körper tragbaren Gehäuse (2), das eine Magnetspule (10) auf einem Magnet-Eisenkern (9) zur Felderzeugung, eine Elektronik auf einer Elektronikplatine (8) für eine Impulserzeugung und eine Hochleistungsbatterie (11) zur Stromversorgung enthält,
wobei der Magnet-Eisenkern (9) als Hohlkörper aus elektrisch leitfähigem Material ausgebildet ist und die Magnetspule (10) auf den Magnet-Eisenkern-Hohlkörper (9) gewickelt ist,
**dadurch gekennzeichnet,**
**dass** die Elektronikplatine (8) mit den Elektronikbauteilen (12) zumindest teilweise im Magnet-Eisenkern-Hohlkörper (9) aufgenommen ist.

2. Magnetfeldgenerator nach Anspruch 1, **dadurch gekennzeichnet, dass** der Eisenkern-Hohlkörper ein beidseitig offenes Eisenkern-Blechrohr (9) bestimmter Länge ist, in das eine längere Elektronik-Platine (8) mit beidseitigen Platinen-Überständen (17, 18) eingesetzt ist.

3. Magnetfeldgenerator nach Anspruch 2, **dadurch gekennzeichnet, dass** an einem Platinen-Überstand (17) ein durch einen Durchbruch des Gehäuses (2) ragender Schalter (3), vorzugsweise als Drehschalter-Nippel, zur Aktivierung und Deaktivierung des Naturfeldsimulators (1) angeordnet ist, der insbesondere eine Bohrung (4) zur Befestigung an einem Halsband aufweist, und/oder
dass am anderen Platinen-Überstand (18) eine betriebsanzeigende Leuchtdiode (5) angeordnet ist, die hinter einer transparent abgedeckten, vorzugsweise durch einen Kristall abgedeckten Gehäuseöffnung als Lichtaustrittsöffnung liegt und dieser Platinen-Überstand (18) endseitig insbesondere konkav gegebenenfalls mit Kontaktfedern zur flächengleichen Aufnahme einer Knopfbatterie (11) als Hochleistungsbatterie ausgebildet ist.

4. Magnetfeldgenerator nach einem der Ansprüche 1 bis 3, **dadurch gekennzeichnet, dass** der Eisenkern-Hohlkörper als Eisenkern-Blechrohr (9) aus zwei vorzugsweise flachen Blech-Schalenteilen (13, 14) gebildet ist, deren jeweils einander gegenüberliegende Schalenlängsränder durch wenigstens einen in Längsrichtung zum Magnetfeld verlaufenden isolierenden Spalt (16) beabstandet sind.

5. Magnetfeldgenerator nach Anspruch 4, **dadurch gekennzeichnet, dass** die Elektronik-Platine (8) seitlich abstehende Nocken (15) als Platinen-Fortsätze aufweist, die jeweils zwischen die Schalenlängsränder zur Realisierung der Spalte (16) ragen, und
dass die Schalenränder im Nockenbereich jeweils eine Haltekontur für die dazwischen liegenden Nocken (15) zur Fixierung der Elektronik-Platine (8) aufweisen, wobei die Schalenteile (13, 14) über die Elektronik-Platine (9) verbunden, insbesondere dort verlötet sind.

6. Magnetfeldgenerator nach Anspruch 4 oder Anspruch 5, **dadurch gekennzeichnet, dass** die zwei Blechschalenteile (13, 14) Gleichteile sind.

7. Magnetfeldgenerator nach einem der Ansprüche 1 bis 6, **dadurch gekennzeichnet, dass** der Magnet-Eisenkern als Hohlkörper (9) mit zumindest teilweise darin aufgenommener Elektronik-Platine (8) eine Baugruppe (6) bildet, die auch den Wickelkörper für die Magnetwicklung der Magnetspule (10) bildet.

8. Magnetfeldgenerator nach einem der Ansprüche 1 bis 7, **dadurch gekennzeichnet, dass** die Hochleistungsbatterie eine Lithium-Knopfbatterie (11) ist, die aus einer magnetisch leitenden Metall-Legierung besteht und in Richtung der magnetischen Achse der Magnetspule (10) angeordnet ist.

9. Magnetfeldgenerator nach einem der Ansprüche 1 bis 8, **dadurch gekennzeichnet, dass** das Gehäuse (2) eine durch eine Verschlusskappe (7) lösbar abgedeckte Öffnung als Batteriefach aufweist, durch die bei der Montage auch die Baugruppe (6) einführbar und fixierbar ist.

## Claims

1. Magnetic field generator as a natural field simulator (1) in a housing (2) which can be worn on the body and which contains a magnet coil (10) on a magnet iron core (9) for field generation, an electronics system on an electronics printed circuit board (8) for pulse generation and a high-power battery (11) for power supply,
wherein the magnet iron core (9) is designed as a hollow body composed of electrically conductive material and the magnet coil (10) is wound onto the magnet iron core hollow body (9),
**characterized in that**
the electronics printed circuit board (8), together with the electronics components (12), is at least partially received in the magnet iron core hollow body (9).

2. Magnetic field generator according to Claim 1, **characterized in that** the iron core hollow body is an iron core sheet-metal tube (9) of specific length which is open on both sides and into which a relatively long electronics printed circuit board (8) with printed circuit board protrusions (17, 18) on both sides is inserted.

3. Magnetic field generator according to Claim 2, **characterized in that** a switch (3) which protrudes through an aperture of the housing (2), preferably as a rotary switch knob, for activating and deactivating the natural field simulator (1) and which has, in particular, a bore (4) for fastening to a necklace is arranged on a printed circuit board protrusion (17), and/or
**in that** an operation-indicating light-emitting diode (5) is arranged on the other printed circuit board projection (18), which operation-indicating light-emitting diode is situated behind a transparently covered housing opening, which is preferably covered by a crystal, as a light exit opening and this printed circuit board protrusion (18) is designed at the end side, in particular concavely, possibly with contact springs for coextensively receiving a button battery (11) as the high-power battery.

4. Magnetic field generator according to one of Claims 1 to 3, **characterized in that** the iron core hollow body is formed as an iron core sheet-metal tube (9) composed of two preferably flat sheet-metal shell parts (13, 14), the mutually opposite shell longitudinal edges of which are spaced apart by at least one insulating gap (16) which runs in the longitudinal direction to the magnetic field.

5. Magnetic field generator according to Claim 4, **characterized in that** the electronics printed circuit board (8) has laterally protruding cams (15) as printed circuit board projections which each protrude between the shell longitudinal edges for realizing the gap (16), and
**in that** the shell edges in the cam region each have a holding contour for the cams (15) situated therebetween for fixing the electronics printed circuit board (8), wherein the shell parts (13, 14) are connected by means of the electronics printed circuit board (9), in particular soldered there.

6. Magnetic field generator according to Claim 4 or Claim 5, **characterized in that** the two sheet-metal shell parts (13, 14) are identical parts.

7. Magnetic field generator according to one of Claims 1 to 6, **characterized in that** the magnet iron core as hollow body (9) with the electronics printed circuit board (8) at least partially accommodated therein forms an assembly (6) which also forms the winding body for the magnet winding of the magnet coil (10).

8. Magnetic field generator according to one of Claims 1 to 7, **characterized in that** the high-power battery is a lithium button battery (11) which is composed of a magnetically conductive metal alloy and is arranged in the direction of the magnetic axis of the magnet coil (10).

9. Magnetic field generator according to one of Claims 1 to 8, **characterized in that** the housing (2) has an opening, which is releasably covered by a closure cap (7), as a battery compartment through which the assembly (6) can also be introduced and fixed during mounting.

## Revendications

1. Générateur de champ magnétique, en tant que simulateur de champ naturel (1), dans un boîtier (2) pouvant être porté sur le corps et renfermant une bobine magnétique (10) sur un noyau ferromagnétique (9) pour la génération d'un champ, une électronique sur une carte de circuit imprimé électronique (8) pour la génération d'impulsions, et une batterie haute performance (11) pour l'alimentation électrique,
dans lequel le noyau ferromagnétique (9) est réalisé sous la forme d'un corps creux constitué de matériau électriquement conducteur et la bobine magnétique (10) est enroulée sur le corps creux du noyau ferromagnétique (9), **caractérisé en ce que** la carte de circuit imprimé électronique (8) munie des composants électroniques (12) est au moins partiellement logée dans le corps creux (9) du noyau ferromagnétique.

2. Générateur de champ magnétique selon la revendication 1, **caractérisé en ce que** le corps creux du noyau de fer est un tube en tôle de noyau de fer (9), ouvert des deux côtés et ayant une longueur déterminée, dans lequel est inséré une carte de circuit imprimé électronique (8) plus longue présentant des parties saillantes (17, 18) des deux côtés de la carte de circuit imprimé.

3. Générateur de champ magnétique selon la revendication 2, **caractérisé en ce qu'**un commutateur (3), de préférence sous la forme d'un embout de commutateur tournant, pour activer et désactiver le simulateur de champ naturel (1), qui présente en particulier un alésage (4) pour la fixation à un collier, est disposé sur une partie saillante (17) de la carte de circuit imprimé en dépassant d'une ouverture traversante du boîtier (2), et/ou **en ce que**, sur l'autre partie saillante (18) de la carte de circuit imprimé, est disposée une diode électroluminescente d'indication de fonctionnement (5), qui est située à l'arrière d'une ouverture de boîtier transparente recouverte, de préférence recouverte d'un cristal, en tant qu'ouverture d'émission de lumière, et **en ce que** ladite partie saillante (18) de la carte de circuit imprimé est réalisée du côté de l'extrémité, notamment de manière concave, le cas échéant avec des ressorts de contact permettant de loger de manière coextensive une batterie bouton (11) en tant que batterie haute performance.

4. Générateur de champ magnétique selon l'une des revendications 1 à 3, **caractérisé en ce que** le corps creux de noyau de fer est réalisé sous la forme d'un tube de tôle de noyau de fer (9) à partir de deux parties de coque en tôle (13, 14) de préférence plates, dont les bords longitudinaux opposés respectifs sont séparés par au moins un interstice isolant (16) s'étendant dans la direction longitudinale du champ magnétique.

5. Générateur de champ magnétique selon la revendication 4, **caractérisé en ce que** la carte de circuit imprimé électronique (8) présente des cames (15) qui s'écartent latéralement en tant que prolongements de la carte de circuit imprimé électronique, qui font respectivement saillie entre les bords longitudinaux de la coque pour réaliser l'interstice (16), et **en ce que** les bords de la coque présentent respectivement, dans la zone des cames, un contour de maintien des cames (15) situées entre ceux-ci pour la fixation de la carte de circuit imprimé électronique (8), dans lequel les parties de coque (13, 14) sont reliées par l'intermédiaire de la carte de circuit imprimé électronique (9), et y sont notamment soudées.

6. Générateur de champ magnétique selon la revendication 4 ou 5, **caractérisé en ce que** les deux parties de coque en tôle (13, 14) sont des pièces identiques.

7. Générateur de champ magnétique selon l'une des revendications 1 à 6, **caractérisé en ce que** le noyau ferromagnétique, en tant que corps creux (9), forme, avec la carte de circuit imprimé électronique (8) au moins partiellement montée dans celui-ci, un module (6) qui constitue également le corps d'enroulement de l'enroulement magnétique de la bobine magnétique (10).

8. Générateur de champ magnétique selon l'une des revendications 1 à 7, **caractérisé en ce que** la batterie haute performance est une batterie bouton au lithium (11) qui est constituée d'un alliage métallique magnétiquement conducteur et est disposée dans la direction de l'axe magnétique de la bobine magnétique (10).

9. Générateur de champ magnétique selon l'une des revendications 1 à 8, **caractérisé en ce que** le boîtier (2) présente une ouverture recouverte par un couvercle de fermeture (7) amovible, en tant que compartiment pour batterie, et par l'intermédiaire de laquelle le module (6) peut également être inséré et fixé lors du montage.
